# EUROPEAN PATENT APPLICATION

(11) **EP 3 524 257 A1**
(43) Date of publication of application: **14.08.2019**
(21) Application number: 17860141.5
(22) Date of filing: 30.09.2017
(51) Int. Cl.: A61K 38/08, A61K 38/10, A61K 38/00, A61P 29/00

(54) **USE OF EXCITATORY NERVE INJURY-RELATED POLYPEPTIDE IN PREVENTING, ALLEVIATING OR TREATING PAIN**

(30) Priority: 10.10.2016 CN 201610885295
(71) Applicant: Biocells (Beijing) Biotech Co., Ltd., Beijing 100070 (CN)
(72) Inventor: HAN, Huamin, Beijing 100070 (CN); TIAN, Yujia, Beijing 100070 (CN); TONG, Wei, Beijing 100070 (CN)
(74) Representative: Mewburn Ellis LLP
(86) International application number: PCT/CN2017/104750
(87) International publication number: WO 2018/068670

(57) **Abstract**

There is provided in the present application use of a peptide comprising the amino acid sequence YEKLLDTEI or a functional variant thereof, or a pharmaceutical composition comprising the peptide in the manufacture of a medicament for preventing, alleviating or treating pain, in particular nociceptive pain, and a method for preventing, alleviating or treating pain.

## Description

### REFERENCE TO RELATED APPLICATIONS

The present application claims priority to Chinese Patent Application No. 201610885295.4, filed on October 10, 2016, the entire contents of which are hereby incorporated herein by reference.

### FIELD OF THE INVENTION

The present application generally relates to the field of medicine. In particular, the present application provides use of neuroexcitatory injury-related polypeptides for preventing, alleviating or treating pain.

### BACKGROUND OF THE INVENTION

Pain is an unpleasant feeling that people often suffer in their lifetime. On one hand, pain provides a warning signal that the body is under attack and a special protection function that is necessary in life. On the other hand, pain is the commonest symptom of various diseases and one of the severest problems that jeopardize human health up to now. Pain involves neural activities with very complicated mechanisms. Studies on pain have become one of the important spots in current neuroscience research.

Current studies have shown that the NR2B subunit of the receptor of N-methyl-D-aspartate (NMDA) is involved in physiopathologic processes such as excitatory synaptic transmission in spinal cord neurons, central sensitization and chronic pain. The neurons of dorsal root ganglion (DRG) bear peripheral and central synapses. Injury stimulation is directly received by the terminals of peripheral synapses, and then transmitted to the terminals of central synapses at the superficial lamina of spinal dorsal horn through Aδ and C fibers, thereby resulting in release of neurotransmitters, such as glutamate, aspartate, SP, and CGRP. Glutamate then binds to the NMDA and AMPA receptors. Since Mg²⁺ blocks the NMDA receptor ion channel, only the AMPA receptor is activated and the activated AMPA receptor continues to activate Na⁺ influx into cells, leading to depolarization of membranes, elimination of the blocking of the channel by Mg²⁺, and increase in Ca²⁺ permeability. A large amount of Ca²⁺ permeating into cells activates PKC, which phosphorylates the NMDA receptor, thereby altering the expression ratio of various NMDA receptor subtypes, and simultaneously converting the silent synapses of the NMDA receptor into functional synapses. NMDA-associated NOS is affected by Ca²⁺ concentration. NOS catalyzes enzymatic reaction at the PDZ sites of PSD-95 and PSD-93, resulting in generation of NO. NO activates ornithine cyclase, thereby leading to synthesis of guanylic acid. Guanylic acid in turn enhances calmodulin-dependent phosphorylation of PSD, regulates the release of synaptic transmitters and alters the excitability of neurons. This feedback amplification effect further transmits injury information to the CNS, causing pain sensitivity and amplification of pain signals.

During the above process, it can be seen that the Ca²⁺ concentration and the interaction between NR2B and PSD-95 are critical elements in amplification of pain signal, and therefore the pain signal may be attenuated by limiting one of the elements. Consequently, development of PSD95 inhibitors is of great significance for the treatment of pain.

### SUMMARY OF THE INVENTION

In a first aspect, there is provided in the present application use of a peptide comprising the amino acid sequence YEKLLDTEI (SEQ ID NO: 1) or a functional variant thereof, or a pharmaceutical composition comprising the peptide in the manufacture of a medicament for preventing, alleviating or treating pain in a subject.

In a second aspect, there is provided in the present application a method of preventing, ameliorating or treating pain, comprising administering to a subject in need thereof a peptide comprising the amino acid sequence YEKLLDTEI or a functional variant thereof or a pharmaceutical composition comprising the peptide.

In some embodiments of the use in the first aspect or the method in the second aspect, the functional variant is a variant generated by one or more conservative substitutions of the LDTEI segment in YEKLLDTEI.

In some embodiments, the conservative substitution is selected from the group consisting of a substitution between D and E, a substitution among L, V and I, and a substitution between T and S.

In some embodiments, the functional variant is a variant generated by replacing the LDTEI segment in YEKLLDTEI with a sequence selected from the group consisting of LDTEL, LDTEV, LDTDI, LDTDL, LDTDV, LDSEI, LDSEL, LDSEV, LDSDI, LDSDL, LDSDV, LETEI, LETEL, LETEV, LETDI, LETDL, LETDV, VDTEI, VDTEL, VDTEV, VDTDI, VDTDL, VDTDV, IDTEI, IDTEL, IDTEV, IDTDI, IDTDL, IDTDV, IETEI, IETEL, IETEV, IETDI, IETDL, and IETDV.

In some embodiments of the use in the first aspect or the method in the second aspect, the peptide is a chimeric peptide comprising the amino acid sequence YEKLLDTEI or a functional variant thereof and an internalization peptide.

In some embodiments, the internalization peptide comprises the amino acid sequence YGRKKRRQRRR (SEQ ID NO: 2).

In some embodiments, the chimeric peptide comprises the amino acid sequence YGRKKRRQRRRYEKLLDTEI (SEQ ID NO: 3).

In some embodiments of the use in the first aspect or the method in the second aspect, the pain is nociceptive pain.

In some embodiments, the pain is caused by external stress or stimulus.

In some embodiments, the external stress or stimulus is a thermal stimulus or touch.

In some embodiments, the nociceptive pain is somatic pain and/or visceral pain.

In some embodiments, the somatic pain involves burns, cuts, contusions, shoulder pain, limb pain, backache, arthritis, tendonitis, or fasciitis.

In some embodiments, visceral pain involves stomach pain, appendicitis, pancreatitis, or peptic ulcer.

In some embodiments, the subject in the aforementioned use and methods is a subject without nerve damage.

In some embodiments, the subject is a healthy subject.

### BRIEF DESCRIPTION OF THE DRAWINGS

Figure 1 shows the result of a pull-down assay to detect interaction between P5 and PDZ1/2 domain. M represents a DNA molecular weight marker; Lane 1 shows His+PDZ1/2+P5; Lane 2 shows P5 alone; Lane 3 shows His+P5; and Lane 4 shows His+PDZ1/2. The eluted band shown in Lane 1 contains both P5 and PDZ1/2, confirming that P5 is capable of binding to PDZ1/2 domain.
Figure 2 is a graph showing the results obtained by treating rats with Polypeptide P5, a negative control (normal saline), and a positive control (ibuprofen) in a hot plate experiment.
Figure 3 is a graph showing the results obtained by treating rats with Polypeptide P5, a negative control (normal saline), and a positive control (ibuprofen) in a needling experiment.

### DETAILED DESCRIPTION OF THE INVENTION

The inventors of the present application have established multiple targets for pain treatment by studying the mechanism of pain, designed and screened peptides for preventing, alleviating or treating pain by experiments, and finally obtained peptides having desired properties. The advantages of the peptides obtained in the present application include high efficiency and low toxicity.

Unless otherwise indicated, all the terms used in the present application have the meaning as commonly understood by one of ordinary skill in the art.

The term "chimeric peptide" means a peptide having two peptide components which are not naturally associated with each other. The two peptide components can form a fusion protein or can be linked by a chemical bond.

The term "functional variant" refers to a variant having same or similar biological function and properties as its parent. As a non-limiting example, a "functional variant" can be obtained by performing one or more conservative substitutions in the parent.

The term "PDZ domain" refers to a modular protein domain of approximately 90 amino acids characterized by a high sequence identity (e.g., at least 60%) to a synaptic protein PSD-95, a Drosophila separating connexin Discs-Large (DLG), and an epithelial tight junction protein Z01. The PDZ domain is also known as Discs-Large homolog repeats ("DHRs") and GLGF repeats. The PDZ domain typically has retained a core consensus sequence (Doyle, D. A., 1996, Cell 85: 1067-76). Exemplary PDZ domain-containing proteins and PDZ domain sequences are disclosed in U.S. Application No. 10/714,537.

The term "specific binding" refers to binding between two molecules (e.g., a ligand and a receptor) characterized by one molecule (e.g., a ligand) being capable of binding to another specific molecule (e.g., a receptor) even in the presence of many other different molecules, i.e. the ability of one molecule to preferentially bind to another molecule in a heterogeneous molecule mixture. The specific binding of a ligand to a receptor can also confirm where the binding of a detectably labeled ligand to a receptor is reduced when excess unlabeled ligands are present (i.e., a binding competition assay).

In a first aspect, there is provided in the present application use of a peptide comprising the amino acid sequence YEKLLDTEI (SEQ ID NO: 1) or a functional variant thereof, or a pharmaceutical composition comprising the peptide in the manufacture of a medicament for preventing, alleviating or treating pain in a subject.

In some embodiments, the functional variant is a variant generated by one or more conservative substitutions of the LDTEI segment in YEKLLDTEI.

In some embodiments, the conservative substitution is selected from the group consisting of a substitution between D and E, a substitution among L, V and I, and a substitution between T and S.

In some more particular embodiments, the functional variant is a variant generated by replacing the LDTEI segment of SEQ ID NO: 1 with a sequence selected from the group consisting of LDTEL, LDTEV, LDTDI, LDTDL, LDTDV, LDSEI, LDSEL, LDSEV, LDSDI, LDSDL, LDSDV, LETEI, LETEL, LETEV, LETDI, LETDL, LETDV, VDTEI, VDTEL, VDTEV, VDTDI, VDTDL, VDTDV, IDTEI, IDTEL, IDTEV, IDTDI, IDTDL, IDTDV, IETEI, IETEL, IETEV, IETDI, IETDL, and IETDV.

In some embodiments, the functional variants disclosed herein also comprise an amino acid sequence having at least 60%, 70%, 75%, 80%, 85%, 90%, 95%, or even higher identity to the peptides as mentioned above. It is known in the art that "identity" between two proteins can be determined by aligning the amino acid sequence of a first protein with the sequence of a second protein which comprises a conservative amino acid substitution relative to the first protein. The degree of identity between two proteins can be determined using computer algorithms and methods well-known to those skilled in the art. The identity between two amino acid sequences is preferably determined by using the BLASTP algorithm.

In some embodiments, the functional variants disclosed herein include those having substitutions, deletions, additions and/or insertions of amino acid residues at 1, 2, 3, 4, 5 or more positions as compared with the peptides as mentioned above, thereby differing from the particular peptides disclosed above.

As described above, a functional variant can differ from a particular peptide disclosed above in one or more substitutions, deletions, additions, and/or insertions. Such variants may be naturally occurring or synthetically produced. For example, one or more of the above-described peptide sequences disclosed herein can be modified and their biological activities can be evaluated following any of a variety of techniques well-known in the art as described herein.

In some embodiments, the peptide comprising the amino acid sequence YEKLLDTEI (SEQ ID NO: 1) or a functional variant thereof is a chimeric peptide comprising the amino acid sequence YEKLLDTEI or a functional variant thereof and an internalization peptide.

An "internalization peptide", also known as a cell-penetrating peptide, is widely used in the field of protein drugs and functions to facilitate the uptake and absorption of an active peptide bound thereto by cells. As a non-limiting example, an internalization peptide can be a Tat peptide. One non-limiting example of Tat peptides is YGRKKRRQRRR (SEQ ID NO: 2).

In some embodiments, the internalization peptide comprises the amino acid sequence YGRKKRRQRRR (SEQ ID NO: 2).

In some embodiments, the chimeric peptide comprises the amino acid sequence YGRKKRRQRRRYEKLLDTEI (SEQ ID NO: 3).

It will be understood by those skilled in the art that the peptides described above can optionally be derivatized (e.g., acetylated, phosphorylated, and/or glycosylated) to promote affinity for targets, to facilitate its ability to be transported across cell membrane, or promote stability.

Pain is an unpleasant feeling or emotional experience associated with actual or potential tissue damage. According to the pathophysiology of pain, pain can be divided into nociceptive pain, non-nociceptive pain and total pain, in which nociceptive pain includes somatic pain and visceral pain, and non-nociceptive pain includes neuropathic pain, central pain, peripheral pain and psychological pain.

In some embodiments, the pain to be prevented, alleviated or treated in the present application is nociceptive pain. Nociceptive pain is a physiological response caused by activation and sensitization of nociceptors, such as Aδ and C-type nerve fibers, by injure stimulus. The response is a pathophysiological process of generation of tissue damage. Various factors produced during tissue damage, such as H⁺, prostaglandins, bradykinin, 5-serotonin, and adenosine, stimulate the sensory nerve terminals, thereby sensitizing various nerve terminal receptors, causing transmission of various pain signals along the nerve fibers, and generating a painful feeling.

In some embodiments, the pain to be prevented, alleviated or treated in the present application is caused by external stress or stimulus. In some embodiments, the external stress or stimulus is a thermal stimulus or touch.

In some embodiments, the pain to be prevented, alleviated or treated in the present application is somatic pain. Somatic pain is caused by pain receptors at the surface (skin tissues) or deep tissues (skeletal and muscle tissues) receiving various injure stimulus. The former is also known as superficial somatic pain, and the latter as deep somatic pain.

In some embodiments, somatic pain involves, but not limited to, burns, cuts, contusions, shoulder pain, limb pain, backache, arthritis, tendinitis, or fasciitis.

In some embodiments, the nociceptive pain to be prevented, alleviated or treated in the present application is visceral pain. Visceral pain is caused by activation of pain receptors in chest, abdomen, or pelvic organs due to penetration, compression, stretching or twisting in these regions, and is often difficult to be located.

In some embodiments, visceral pain involves, but not limited to, stomach pain, appendicitis, pancreatitis, or peptic ulcer.

In some embodiments, a pharmaceutical composition comprising a peptide comprising the amino acid sequence YEKLLDTEI or a functional variant thereof can be prepared by conventional methods of mixing, dissolving, granulating, tableting, milling, emulsifying, encapsulating, capturing or lyophilizing.

In some embodiments, the pharmaceutical composition can be formulated in a conventional manner using one or more physiologically acceptable carriers, diluents, excipients, or auxiliary materials.

In some embodiments, the peptide or pharmaceutical composition can be formulated in an aqueous solution, preferably in a physiologically compatible buffer such as Hank's solution, Ringer's solution, or physiological saline or acetate buffer (to reduce discomfortableness at injection sites). The solution may contain formulating agents such as suspending, stabilizing and/or dispersing agents.

In some embodiments, the peptide or pharmaceutical composition of the present application may be in the form of a powder for re-constitution with a suitable carrier, such as sterile non-pyrogenic water, prior to use.

In some embodiments, penetrants appropriate for penetrating the barrier of interest are used in the formulation.

In some embodiments, the peptide of the present application can be formulated with a pharmaceutically acceptable carrier into tablets, pills, troches, capsules, liquids, gels, syrups, slurries, suspensions or the like. For solid formulations such as powders, capsules and tablets, suitable excipients include fillers such as sugars such as lactose, sucrose, mannitol and sorbitol; cellulose preparations such as corn starch, wheat starch, rice starch, potato starch, gelatin, tragacanth, methylcellulose, carboxypropylmethylcellulose, sodium carboxymethylcellulose and/or povidone (PVP); granulating agents and binders. If necessary, a disintegrating agent, such as crosslinked polyvinylpyrrolidone, agar, or alginic acid or a salt thereof (such as sodium alginate), may be added. If necessary, the solid formulations can be coated with sugar or enteric coating using standard techniques. For oral liquid preparations such as suspensions, elixirs and solutions, suitable carriers, excipients or diluents include water, glycerol, oil and alcohol. Furthermore, a flavoring agent, a preservative, a coloring agent, or the like may be added.

In addition to the formulations as described above, the peptide of the present application can also be formulated into a reservoir preparation. Thus, for example, the compound can be formulated with a suitable polymeric or hydrophobic material (for example, formulated as an emulsion in an acceptable oil) or an ion exchange resin, or formulated as a sparingly soluble derivative, for example, a sparingly soluble salt.

In some embodiments, the subject as mentioned above is a subject without nerve damage. In some embodiments, the subject is a healthy subject.

In a second aspect, there is provided in the present application a method of preventing, ameliorating or treating pain, comprising administering to a subject in need thereof a peptide comprising the amino acid sequence YEKLLDTEI or a functional variant thereof or a pharmaceutical composition comprising the peptide.

In some embodiments, the functional variant is a variant generated by one or more conservative substitutions of the LDTEI segment in YEKLLDTEI. In some embodiments, the conservative substitution is selected from the group consisting of a substitution between D and E, a substitution among L, V and I, and a substitution between T and S. In some more particular embodiments, the functional variant is a variant generated by replacing the LDTEI segment of SEQ ID NO: 1 with a sequence selected from the group consisting of LDTEL, LDTEV, LDTDI, LDTDL, LDTDV, LDSEI, LDSEL, LDSEV, LDSDI, LDSDL, LDSDV, LETEI, LETEL, LETEV, LETDI, LETDL, LETDV, VDTEI, VDTEL, VDTEV, VDTDI, VDTDL, VDTDV, IDTEI, IDTEL, IDTEV, IDTDI, IDTDL, IDTDV, IETEI, IETEL, IETEV, IETDI, IETDL, and IETDV.

In some embodiments, the functional variants disclosed herein also comprise an amino acid sequence having at least 60%, 70%, 75%, 80%, 85%, 90%, 95%, or even higher identity to the peptides as mentioned above. It is known in the art that "identity" between two proteins can be determined by aligning the amino acid sequence of a first protein with the sequence of a second protein which comprises a conservative amino acid substitution relative to the first protein. The degree of identity between two proteins can be determined using computer algorithms and methods well-known to those skilled in the art. The identity between two amino acid sequences is preferably determined by using the BLASTP algorithm.

In some embodiments, the functional variants disclosed herein include those having substitutions, deletions, additions and/or insertions of amino acid residues at 1, 2, 3, 4, 5 or more positions as compared with the peptides as mentioned above, thereby differing from the particular peptides disclosed above.

As described above, a functional variant can differ from a particular peptide disclosed above in one or more substitutions, deletions, additions, and/or insertions. Such variants may be naturally occurring or synthetically produced. For example, one or more of the above-described peptide sequences disclosed herein can be modified and their biological activities can be evaluated following any of a variety of techniques well-known in the art as described herein.

In some embodiments, the peptide comprising the amino acid sequence YEKLLDTEI (SEQ ID NO: 1) or a functional variant thereof is a chimeric peptide comprising the amino acid sequence YEKLLDTEI or a functional variant thereof and an internalization peptide.

An "internalization peptide", also known as a cell-penetrating peptide, is widely used in the field of protein drugs and functions to facilitate the uptake and absorption of an active peptide bound thereto by cells. As a non-limiting example, an internalization peptide can be a Tat peptide. One non-limiting example of Tat peptides is YGRKKRRQRRR (SEQ ID NO: 2).

In some embodiments, the internalization peptide comprises the amino acid sequence YGRKKRRQRRR (SEQ ID NO: 2).

In some embodiments, the chimeric peptide comprises the amino acid sequence YGRKKRRQRRRYEKLLDTEI (SEQ ID NO: 3).

It will be understood by those skilled in the art that the peptides described above can optionally be derivatized (e.g., acetylated, phosphorylated, and/or glycosylated) to promote affinity for targets, to facilitate its ability to be transported across cell membrane, or promote stability.

In some embodiments, the pain is nociceptive pain.

In some embodiments, the pain is caused by a thermal stimulus or touch.

In some embodiments, the pain is caused by heating or punching.

In some embodiments, the nociceptive pain to be prevented, ameliorated or treated is somatic pain. In some embodiments, somatic pain involves, but not limited to, burns, cuts, contusions, shoulder pain, limb pain, backache, arthritis, tendinitis, or fasciitis.

In some embodiments, the nociceptive pain to be prevented, alleviated or treated is visceral pain. In some embodiments, visceral pain involves, but not limited to, stomach pain, appendicitis, pancreatitis, or peptic ulcer.

In some embodiments, administration of a peptide comprising the amino acid sequence YEKLLDTEI or a functional variant thereof, or a pharmaceutical composition comprising the peptide, can be parenteral, intravenous, oral, subcutaneous, intraarterial, intracranial, intrathecal, intraperitoneal, topical, intranasal, or intramuscular administration. Intravenous administration is preferred.

In some embodiments, the pharmaceutical composition of the present application for parenteral administration is preferably sterile and substantially isotonic. For injection, the peptide or pharmaceutical composition can be formulated in an aqueous solution, preferably in a physiologically compatible buffer such as Hank's solution, Ringer's solution, or physiological saline or acetate buffer (to reduce discomfortableness at injection sites). The solution may contain formulating agents such as suspending, stabilizing and/or dispersing agents.

Alternatively, the peptide or pharmaceutical composition of the present application may be in the form of a powder for re-constitution with a suitable carrier, such as sterile non-pyrogenic water, prior to use.

For transmucosal administration, penetrants appropriate for penetrating the barrier of interest are used in the formulation. This administration route can be used to deliver a compound to the nasal cavity or for sublingual administration.

In some embodiments, for oral administration, the peptide of the present application can be formulated with a pharmaceutically acceptable carrier into tablets, pills, troches, capsules, liquids, gels, syrups, slurries, suspensions or the like, for oral ingestion by a patient to be treated. For oral solid formulations such as powders, capsules and tablets, suitable excipients include fillers such as sugars such as lactose, sucrose, mannitol and sorbitol; cellulose preparations such as corn starch, wheat starch, rice starch, potato starch, gelatin, tragacanth, methylcellulose, carboxypropylmethylcellulose, sodium carboxymethylcellulose and/or povidone (PVP); granulating agents and binders. If necessary, a disintegrating agent, such as crosslinked polyvinylpyrrolidone, agar, or alginic acid or a salt thereof (such as sodium alginate), may be added. If necessary, the solid formulations can be coated with sugar or enteric coating using standard techniques. For oral liquid preparations such as suspensions, elixirs and solutions, suitable carriers, excipients or diluents include water, glycerol, oil and alcohol. Furthermore, a flavoring agent, a preservative, a coloring agent, or the like may be added.

In addition to the formulations as described above, the peptide of the present application can also be formulated into a reservoir preparation. Such long-acting formulations can be administered by implantation (for example subcutaneous or intramuscular) or by intramuscular injection. Thus, for example, the compound can be formulated with a suitable polymeric or hydrophobic material (for example, formulated as an emulsion in an acceptable oil) or an ion exchange resin, or formulated as a sparingly soluble derivative, for example, a sparingly soluble salt.

Alternatively, other drug delivery systems can be used. The chimeric peptide can be delivered using liposomes and emulsions. Certain organic solvents, such as dimethyl sulfoxide, can also be used. Additionally, a compound can be delivered using a sustained release system, such as a semipermeable substrate of solid polymers containing a therapeutic agent.

The peptide or pharmaceutical composition of the present application is administered in a therapeutically effective amount effective to achieve the intended purpose (e.g., to prevent, alleviate or treat nociceptive pain). A therapeutically effective amount means the amount of the peptide or pharmaceutical composition disclosed herein sufficient to significantly prevent, alleviate or treat pain in subjects (or animal models) treated with the peptide or pharmaceutical composition as compared to pain in a control population of subjects (or animal models) not treated with the peptide or pharmaceutical composition.

In some embodiments, the peptide is administered at a dose ranging from 0.1 µg to 10 µgs/kg, such as 0.1 µg/kg, 0.2 µg/kg, 0.3 µg/kg, 0.4 µg/kg, 0.5 µg/kg, 0.6 µg./kg, 0.7 µg/kg, 0.8 µg/kg, 0.9 µg/kg, 1 µg/kg, 2 µg/kg, 3 µg/kg, 4 µg/kg, 5 µg/kg, 6 µg/kg, 7 µg /kg, 8 µg/kg, 9 µg/kg, or 10 µg/kg. In some embodiments, the preferred dose is 0.2 µg/kg.

In some embodiments, the administered amount of the peptide or the pharmaceutical composition of the present application depends on the subject being treated, the weight of the subject, the severity of pain, the administration route, and the adjustments by the prescribing physician.

In some embodiments, a therapeutically effective dose of the peptide or the pharmaceutical composition of the present application is capable of providing a therapeutic benefit without causing significant toxicity. The toxicity can be determined in cell cultures or experimental animals by standard pharmaceutical procedures, for example by determining LDso (a dose that kills 50% of the population) or LD₁₀₀ (a dose that kills 100% of the population). The dose ratio between toxic effect and therapeutic effect is the therapeutic index. The experiment demonstrated that the peptide of the present application had no significant toxicity at a dose of 200 mg/kg.

In some embodiments, the efficacy of the peptide or composition of the present application can last for at least two hours.

In some embodiments, the aforementioned subject is a subject without nerve damage.

In some embodiments, the subject is a healthy subject.

It should be understood that the foregoing detailed description only aims to help those skilled in the art to more clearly understand the present application, but is not intended to limit the present application in any way. Those skilled in the art can make various modifications and changes to the described embodiments.

### EXAMPLES

The following examples are provided only to illustrate some embodiments of the present application without any purpose or nature of limitation.

### Example 1: Screening of active peptide molecules

Based on reported study results, the Tat transmembrane peptide YGRKKRRQRRR was selected and ligated to various numbers of amino acids to form a peptide library. The peptide molecules in the peptide library were tested for interaction with the PDZ1/2 domain expressed and purified *in vitro,* and the polypeptides were preliminarily screened for the strength of interaction force.

The immobile phase molecule (ligand) was PDZ1/2 protein with a molecular weight of approximately 20kD at a concentration of 2mg/ml. The mobile phase molecule (analyte) was a polypeptide to be screened with a molecular weight of approximately 2 kD at a concentration of 10mg/ml. The CM5 chip was used for fixation using a Biacore 3000 instrument. The electrophoresis buffer was PBS plus 0.005% Tween 20. Fixation was carried out using an amino coupling method. The concentration of the ligand was 10 µg/ml. The fixation buffer was 10 mM sodium acetate, pH 4.0. Fixed amount was 1400 RU, which was fixed to flow cell 2. The used flow rate was 10 µl/ml and the ligand was loaded for 1 minute. 10 mM Gly at pH 2.0 + 2.5 was used as a regenerant. Regeneration was carried out at a flow rate of 30 µl/min. The loading time was 30 s.

Kinetic analysis was performed using the following conditions.
control channel: flow cell 1;
electrophoresis buffer: PBS;
mode: Kinetic Analysis Wizard;
concentration gradients: 6.25n, 12.5n, 25n, 50n, 100n, 200n, 400nM;
loading time:1 minute;
dissociation time: 2 min; and
flow rate: 30 µl/min.

The data was fitted using the fitting software Biaevaluation 4.1. The fitting model was a 1:1 binding model. The dissociation constant KD value was inversely proportional to the interaction force.

By screening, a chimeric peptide having strong capability of interacting with the PDZ1/2 domain was obtained, and named as P5. The sequence of the chimeric peptide was shown below.
P5: YGRKKRRQRRRYEKLLDTEI
The chimeric peptide P5 was subjected to further testing in the following experiments.

### Example 2: Pull-down assay to verify the interaction of P5 with PDZ1/2 domain

To confirm that P5 can interact with the PDZ1/2 domain, a pull-down assay was performed.

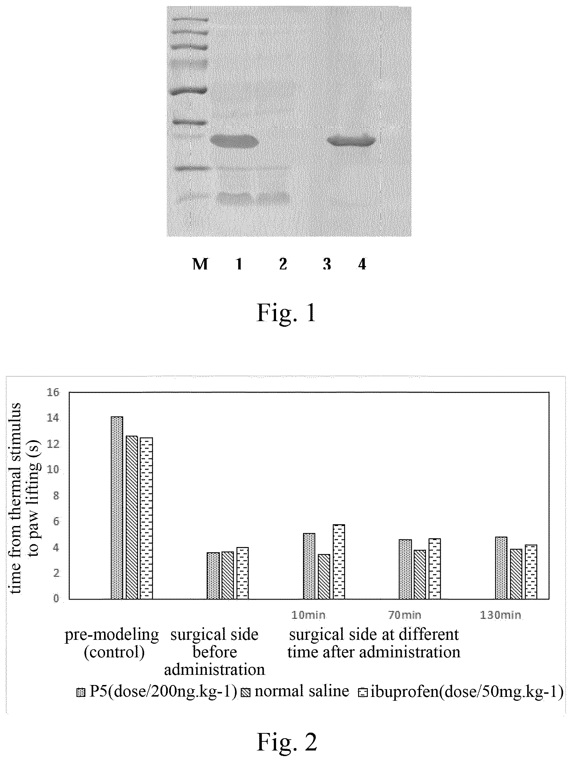

The column was equilibrated with 100 µl of His beads and 1 ml of MCAC-0 buffer for 5 min and shaked at 4 °C. The mixture was centrifuged at 5000 g for 1 minute at 4°C, and the supernatant was discarded. 1 mg of PDZ1/2 protein was added to the mixture, and a buffer was added to reach the volume of 1 ml. The mixture was spun for binding for 1 hour at 4 °C. The mixture was centrifuged at 5000 g for 1 minute at 4 °C, and the supernatant was discarded. The mixture was washed three times with 1 ml of MCAC-0 buffer for 5 minutes each time (at 4 °C, washing with shaking). 1 mg of P5 protein was added to the mixture, and a buffer was added to reach the volume of 1 ml. The mixture was spun for binding for 2 hours at 4 °C. The mixture was centrifuged at 5000 g for 1 minute at 4 °C, and the supernatant was discarded. The mixture was washed three times with 1 ml of lysis buffer for 5 minutes each time (at 4 °C, washing with shaking). 20 µl of MCAC-300 was added after washing. After centrifugation, the eluate was taken for a SDS-PAGE assay. The experimental results were shown in Figure 1.

As shown in Figure 1, both P5 and the PDZ1/2 domain were contained in the eluted band of the polypeptide P5, thereby confirming that the polypeptide P5 can bind to the PDZ1/2 domain.

### Example 3: Evaluation of analgesic effect of Polypeptide P5 in a hot plate experiment

### Experimental Protocols

### Animal Inclusion

Eighteen male SD rats of 240-260 g (purchased from Huafukangcheng Co., Ltd.) were selected as experimental rats, and divided into three groups with six rats in each group for a hot plate experiment, including a group administered with Polypeptide P5 at a dose of 0.2 µg/kg, a group administered with normal saline and a group administered with positive control drug (ibuprofen, 50 mg/kg). Screening was conducted before the experiment. In particular, before the hot plate experiment, the candidate rats were placed on a hot plate at 55 °C, and the rats which did not lick the hind paws or lift paws within 30 seconds were excluded from the experiment.

### Construction of Animal Model

4.0 chromic catguts (purchased from Shandong Boda Medical Products Co., Ltd.) were immersed into normal saline (purchased from Shijiazhuang Four Medicine Co., Ltd., National Pharmaceutical Standard H13023200) for 30 minutes for softening. Rats were shaved in the middle of their thighs, and wiped with iodine. Wound at 1 cm below the epidermis was created with a scissor, and then a J31060 hemostat was used to tear the biceps femoris. The sciatic nerve was thus exposed at the middle of the thighs. The end proximal to the leg was the trigeminal sciatic nerve. The sciatic nerve at the middle of the thighs was gently ligated four times with the 4.0 chromic catguts. The force was deemed appropriate if muscles near the ligation site slightly trembled.

The adjacent ligation sites were 1 mm apart. Then muscles and epidermal tissues were sutured separately.

### Administration

Before the experiment, the rats with successful surgery in the test group were injected via caudal vein with Peptide P5 in normal saline (dose at 0.2 µg/kg). Rats in normal saline group were intraperitoneally injected with 0.3 mL of normal saline. The positive drug group was administered via intragastric infusion with ibuprofen suspension (purchased from Shanghai Qiangsheng Pharmaceutical Co., Ltd., National Pharmaceutical Standard H19991011) at a dose of 50 mg/kg.

### Hot Plate Experiment

Hot plate experiments were performed on rats 7 days after surgery. Rats were feed properly, and the indoor environment was maintained at about 25°C. The temperature of the YLS-6B intelligent hot plate instrument (purchased from Beijing Zhongshi Di Chuang Technology Development Co., Ltd.) was set at about 55°C. The rats were placed on the hot plate instrument in a closed, detachable, and transparent plexiglass jar, and the time of occurrence of the hind paw licking or jump reaction was recorded. If there no reaction was observed from the rats with administration 20 seconds after placement in the jar, the SD rats were taken out to avoid scalding the paws due to too long heating. The pain threshold of such rats was calculated as 20 seconds. Each rat was tested twice in succession with the interval of at least 30 seconds. The average of results was calculated.

At the end of the experiment, the heating pain response time (i.e., pain threshold in second) of rats in the normal saline control group and individual drug-administered groups before administration and 10 min, 70 min, and 130 min after administration was recorded.

### Experimental Results

EXCEL's T-test function (two-tailed variance) was used to analyze the statistical difference between the paw lifting time thresholds due to heating pain of pre-modeling and post-modeling experimental rats to evaluate whether or not the modeling was successful. The difference among the heating pain time thresholds before administration and at each time point after administration was then analyzed to evaluate the pharmaceutical efficacy. P < 0.05 was considered statistically different, and P < 0.01 was considered significantly statistically different.

The experimental results are shown in Table 1 below and Figure 2.

**Table 1 Statistical results of heating pain time thresholds of rats treated with the Polypeptide P5**

| drug dose/mg.kg⁻¹ | pre-modeling (control)(s) | surgical side before administration (s) | paw liftingtime thresholdsduetoheating painatsurgical side ofratsatdifferenttime afteradministration (s) | | |
|---|---|---|---|---|---|
| | | | 10 min | 70 min | 130 min |
| P5(0.0002) | 14.10 | 3.55 | 5.09 | 4.59 | 4.79 |
| normal saline | 12.57 | 3.66 | 3.45 | 3.75 | 3.84 |
| ibuprofen (50) | 12.5 | 3.95 | 5.74 | 4.64 | 4.19 |

The results showed that, for the rats in the treatment group at a dose of 0.2 µg/kg, there was significant statistical difference in paws lifting time threshold at the surgical side between the pre-modeling and post-modeling rats (p=0.0002), indicating that the modeling was successful. There was statistical difference between rats before administration and 10 min after administration at a dose of 0.2 µg/kg (p=0.013). There was statistical difference between rats 10 min after P5 administration and 10 min after normal saline administration (p=0.028), indicating analgesic effect could be produced in a short time after P5 administration. In addition, there was statistical difference between rats before administration and 130 min after P5 administration at a dose of 0.2 µg/kg (p=0.047). There was no statistical difference between rats 10 min after administration and 130 min after administration (p=0.697), indicating that there was no statistical change in analgesia effect from 10 minutes after administration to 130 minutes after administration. That is, the therapeutic effect of the Polypeptide P5 could last for at least 130 minutes.

The results of the positive drug control group showed that, for the rats treated with ibuprofen at a dose of 50 mg/kg, there was statistical difference between the healthy side and the surgical side in paw lifting time threshold (p<0.01), indicating that the modeling was successful. There was statistical difference between rats before administration and 10 min after administration (p=0.03), indicating that analgesic effect can be produced in a short time after ibuprofen administration. However, there was no statistical difference in paw lifting time thresholds at surgical side between rats 130 min after administration and before administration (p>0.05), indicating the therapeutic effect could not last for 130 minutes.

### Example 4: Evaluation of analgesic effect of the Polypeptide P5 in a needling experiment

### Experimental Protocols

### Rat Inclusion

Eighteen male SD rats of 240-260 g (purchased from Huafukangcheng Co., Ltd.) were selected as experimental rats, and divided into three groups with six rats in each group for a needling experiment, including a group administered with the polypeptide at a dose of 0.2 µg/kg, a normal saline group and a positive control drug group (ibuprofen, 50 mg/kg).

### Modeling

### Construction of Animal Model

4.0 chromic catguts (purchased from Shandong Boda Medical Products Co., Ltd.) were immersed into normal saline (purchased from Shijiazhuang Four Medicine Co., Ltd., National Pharmaceutical Standard H13023200) for 30 minutes for softening. Rats were shaved in the middle of their thighs, and wiped with iodine. Wound at 1 cm below the epidermis was created with a scissor, and then a J31060 hemostat was used to tear the biceps femoris. The sciatic nerve was thus exposed at the middle of the thighs. The end proximal to the leg was the trigeminal sciatic nerve. The sciatic nerve at the middle of the thighs was gently ligated four times with the 4.0 chromic catguts. The force was deemed appropriate if muscles near the ligation site slightly trembled.

The adjacent ligation sites were 1 mm apart. Then muscles and epidermal tissues were sutured separately.

### Administration

Before the experiment, the rats with successful surgery in the test group were injected via caudal vein with Peptide P5 in normal saline (dose at 0.2 µg/kg). Rats in normal saline group were intraperitoneally injected with 0.3 mL of normal saline. The positive drug group was administered via intragastric infusion with ibuprofen suspension (purchased from Shanghai Qiangsheng Pharmaceutical Co., Ltd., National Pharmaceutical Standard H19991011) at a dose of 50 mg/kg.

### Needling Experiment

Fourteen days after surgery, the rats in the administration groups were tested before administration and 10min\70min\130min after administration. The experimental rats were placed in a plexiglass observation chamber to acclimate the environment for 5 minutes until the rats no longer looked around. The ZS-CTY foot needling system (purchased from Beijing Zhongshi Di Chuang Technology Development Co., Ltd.) was connected to a win7 32-bit computer. The test software was opened to calibrate the zero point of the instrument, and then calibrate again after a 10g weight was added. The vonFrey probe was used to slowly and gently stimulate the middle parts of the testing hind paws of the rats to observe the paw withdrawal reaction of the rats. If the rats showed a rapid paw withdrawal reaction due to stimulation, they were considered response positive. The paw withdrawal reaction of rats due to physical activity was excluded. The reaction was recorded twice at each time point, and averaged to obtain paw lifting force thresholds (in g) due to needling. The foot needling system (purchased from Beijing Zhongshi Di Chuang Technology Development Co., Ltd.; lot. number: 20160101060001) was adjusted to zero with a 10g weight, there was still a fundamental read fluctuation when the bottom of the rats' paws was needled. The fluctuation was generally between +3 and +5g, which should be subtracted from the final value. If there was still a baseline, it should be removed.

### Experimental Results

EXCEL's T-test function (two-tailed variance) was used to analyze the statistical difference in paw lifting force thresholds due to needling between the untreated side and surgical side in the experimental rats to evaluate whether or not the modeling was successful. The difference among the needling pain force thresholds before administration and at each time point after administration was then analyzed to evaluate the pharmaceutical efficacy and effect duration. P < 0.05 was considered statistically different, and P < 0.01 was considered significantly statistically different.

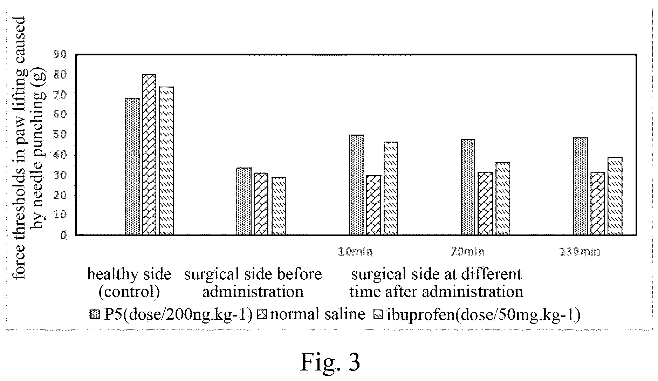

The experimental results are shown in Table 2 below and Figure 3.

**Table 2 Statistical results of needling pain force thresholds of rats treated with the Polypeptide P5**

| drug dose/mg.kg⁻¹ | healthy side (control)/g | at surgical side before administration/g | paw lifting thresholds due to needling at surgicalside of rats at different time after administration /g | | |
|---|---|---|---|---|---|
| | | | 10 min | 70 min | 130 min |
| P5(0.0002) | 68.27 | 33.38 | 49.81 | 47.34 | 48.32 |
| normal saline | 80.10 | 30.86 | 29.52 | 31.44 | 31.19 |
| ibuprofen (50) | 73.65 | 28.72 | 46.24 | 36.21 | 38.88 |

The results showed that, for the rats in the treatment group at a dose of 0.2 µg/kg, there was statistical difference in paw lifting force threshold due to needling between the healthy side and the surgical side (p=0.0004), indicating that the modeling was successful. As for the rats in the treatment group at a dose of 0.2 µg/kg, there was statistical difference in paw lifting force threshold due to needling between rats before administration and 10 min after administration at the surgical side p=0.03. There was statistical difference in paw lifting force threshold due to needling between rats 10 min after P5 administration and 10 min after normal saline administration at the surgical side (p=0.03), indicating that analgesic effect can be produced in a short time after P5 administration. In addition, there was statistical difference in paw lifting force threshold due to needling between rats before administration and 130 min after 0.2 µg/kg dose of P5 administration at the surgical side (p=0.03). The results at 10 min after administration and 130 min after administration, paw lifting force threshold due to needling at the surgical side showed p=0.687, indicating that there was no statistical change in paw lifting force threshold due to needling within 130 minutes after administration. That is, the therapeutic effect of the Polypeptide P5 could last for at least 130 minutes.

|In contrast, the results of the positive control drug group showed that, for rats in the group treated with ibuprofen at a dose of 50 mg/kg, there was statistical difference between the healthy side and the surgical side in paw lifting force threshold due to needling (p<0.01), indicating that the modeling was successful. There was no significant statistical difference between rats before administration and 10 min after administration (p=0.0069). There was no significant statistical difference between rats 10 min after P5 administration and 10 min after normal saline administration (p=0.08). In addition, p>0.05 was obtained between rats 130 min after ibuprofen administration and before administration, indicating that the therapeutic effect of the positive drug could not last for 130 minutes, and therefore the analgesic effect of Polypeptide P5 was superior to the positive drug.

### Example 5: Acute toxicity assessment

Acute toxicity tests were performed on rats. The results showed that P5 had no lethal effect and other obvious toxic side effects on the rats at a dose of 1000 mg/kg body weight.

Various changes and equivalent substitutions can be made to the various embodiments disclosed herein without departing from the spirit and scope of the disclosure. Any feature, step or embodiment of an embodiment of the present disclosure can be used in combination with any other feature, step or embodiment, unless otherwise stated in the context.

## Claims

1. Use of a peptide comprising the amino acid sequence YEKLLDTEI (SEQ ID NO: 1) or a functional variant thereof, or a pharmaceutical composition comprising the peptide in the manufacture of a medicament for preventing, alleviating or treating pain in a subject.

2. A method of preventing, ameliorating or treating pain, comprising administering to a subject in need thereof a peptide comprising the amino acid sequence YEKLLDTEI or a functional variant thereof or a pharmaceutical composition comprising the peptide.

3. The use of claim 1 or the method of claim 2, wherein the functional variant is a variant generated by one or more conservative substitutions of the LDTEI segment in YEKLLDTEI, preferably, the conservative substitution is selected from the group consisting of a substitution between D and E, a substitution among L, V and I, and a substitution between T and S.

4. The use or method of claim 3, wherein the functional variant is a variant generated by replacing the LDTEI segment in YEKLLDTEI with a sequence selected from the group consisting of LDTEL, LDTEV, LDTDI, LDTDL, LDTDV, LDSEI, LDSEL, LDSEV, LDSDI, LDSDL, LDSDV, LETEI, LETEL, LETEV, LETDI, LETDL, LETDV, VDTEI, VDTEL, VDTEV, VDTDI, VDTDL, VDTDV, IDTEI, IDTEL, IDTEV, IDTDI, IDTDL, IDTDV, IETEI, IETEL, IETEV, IETDI, IETDL, and IETDV.

5. The use or method of any of the preceding claims, wherein the peptide is a chimeric peptide comprising the amino acid sequence YEKLLDTEI or a functional variant thereof and an internalization peptide, preferably, the internalization peptide comprises the amino acid sequence YGRKKRRQRRR (SEQ ID NO: 2).

6. The use or method of claim 5, wherein the chimeric peptide comprises the amino acid sequence YGRKKRRQRRRYEKLLDTEI (SEQ ID NO: 3).

7. The use or method of any of the preceding claims, wherein the pain is nociceptive pain, preferably, the pain is caused by external stress or stimulus, more preferably, the external stress or stimulus is a thermal stimulus or touch.

8. The use or method of claim 7, wherein the nociceptive pain is somatic pain and/or visceral pain.

9. The use or method of claim 8, wherein the somatic pain involves burns, cuts, contusions, shoulder pain, limb pain, backache, arthritis, tendonitis, or fasciitis, and/or the visceral pain involves stomach pain, appendicitis, pancreatitis, or peptic ulcer.

10. The use or method of any of the preceding claims, wherein the subject is a subject without nerve damage, preferably, the subject is a healthy subject.
